# EUROPÄISCHE PATENTSCHRIFT

(11) **EP 0 879 815 B1**
(45) Veröffentlichungstag und Bekanntmachung des Hinweises auf die Patenterteilung: **02.11.2000**
(21) Anmeldenummer: 98108385.0
(22) Anmeldetag: 08.05.1998
(51) Int. Cl.: C07C 201/12, C07C 205/57

(54) **Verfahren zur Herstellung von 5-Nitro-isophthalsäuredi-c1-c4-alkylestern**
Process for the preparation of the 5-nitro-isophthalic-di- C1-C4-alkyl esters
Procédé pour la préparation des diesters en alkyle C1-C4 de l'acide nitro-5-isophtalique

(30) Priorität: 21.05.1997 DE 19721221
(43) Veröffentlichungstag der Anmeldung: 25.11.1998
(73) Patentinhaber: BAYER AG, 51368 Leverkusen (DE)
(72) Erfinder: Rauchschwalbe, Günter, Dr., 51375 Leverkusen (DE); Beitzke, Bernhard, Dr., 51503 Rösrath (DE); Fiege, Helmut, Dr., 51373 Leverkusen (DE)

(56) Entgegenhaltungen:
- DE-C- 3 335 312
- R. O. CLINTON ET AL.: "The preparation of methyl esters" JOURNAL OF THE AMERICAN CHEMICAL SOCIETY., Bd. 70, Nr. 9, September 1948, DC US, Seiten 3135-3136, XP002074745
- PATENT ABSTRACTS OF JAPAN vol. 009, no. 130 (C-284), 5.Juni 1985 & JP 60 016953 A (MITSUBISHI KASEI KOGYO KK), 28.Januar 1985,
- H. CASSEBAUM ET AL.: "Dünnflüssige trijodierte Röntgenkontrastmittel für die Angiographie" PHARMAZIE, Bd. 21, Nr. 3, 1966, Seiten 167-170, XP002074746
- A WOHL: "Über Estersäuren und Amidsäuren der Isophthalsäure-Reihe,...." BERICHTE DER DEUTSCHEN CHEMISCHEN GESELLSCHAFT., Bd. 43, 1910, WEINHEIM DE, Seiten 3474-3489, XP002074747

## Beschreibung

5-Nitro-isophthalsäure-dimethylester ist ein wichtiges Zwischenprodukt zur Herstellung von intravenös anzuwendenden Röntgenkontrastmitteln, die als Röntgenstrahlen absorbierende Komponente Derivate der 5-Amino-2,4,6-triiod-isophthalsäure enthalten. Andere 5-Nitro-isophthalsäure-dialkylester können analog verwendet werden. Es besteht ein erheblicher Bedarf an derartigen Zwischenprodukten, die mit möglichst geringem Aufwand und gleichzeitig in hohen Reinheiten zugänglich sein sollten. Auf pharmazeutischem Gebiet müssen bereits Zwischenprodukte im Hinblick auf Nebenkomponenten wenigstens zu 99,5 % rein sein, und eine einzelne Nebenkomponente darf i.a. höchstens zu 0,3 % vorliegen.

Für die Herstellung von 5-Nitro-isophthalsäure-dimethylester sind bereits mehrere Synthesewege bekannt, die jedoch alle nicht befriedigen. Gemäß J. Org. Chem. 26, 2963 (1961) und US-PS 2 680 730 wird Isophthalsäure-dimethylester in konzentrierter oder rauchender Schwefelsäure gelöst und mit einem Gemisch aus Schwefelsäure und Salpetersäure nitriert. Dabei erhält man die gewünschte Verbindung als Hauptkomponente, jedoch enstehen bei der Nitrierung (wie in eigenen Versuchen aufgefunden wurde) nicht unerhebliche Mengen des unerwünschten isomeren 4-Nitroisophthalsäure-dimethylesters. Außerdem entstehen bei der Aufarbeitung durch Austragen des Reaktionsansatzes auf Eis durch Verseifung wechselnde Mengen 5-Nitroisophthalsäure-monomethylester (siehe Beispiel 9). Eine so erhaltene Ware ist für den geschilderten pharmazeutischen Einsatz bei weitem nicht rein genug. Sie muß durch Umkristallisation gereinigt werden, wobei erhebliche Ausbeuteeinbußen auftreten Die o.g. Literatur gibt an, daß das gewünschte Produkt in einer Ausbeute von nur 42 % d.Th. erhalten wird. Selbst das umkristallisierte Produkt ist noch nicht ausreichend rein, wie aus dem Vergleich des Schmelzpunktes dieser Ware (119-121°C) mit dem Schmelzpunkt der reinen Ware (123°C) hervorgeht.

Nach Ber. 43, 3474 (1910) und Pharmazie, 21 (3), 167-170 (1966) kann 5-Nitro-isophthalsäure-dimethylester auch erhalten werden, indem 5-Nitro-isophthalsäure mit einem großen Überschuß an Methanol (beispielsweise 33 Mol Methanol/Mol 5-Nitroisophthalsäure) in Gegenwart von Schwefelsäure verestert wird. Dabei bildet sich zunächst der Monomethylester und daraus dann der Dimethylester, der aus der Lösung auskristallisiert. Allerdings ist das erhaltene Produkt für pharmazeutische Verwendungen nicht rein genug, denn es enthält 0,3 bis 0,5 % des Monomethylesters. Das Produkt muß auch hier unter Inkaufnahme der genannten Nachteile durch Umkristallisation gereinigt werden.

Die Veresterung von 2,4 Dinitrophenylessigsäure unter Verwendung von Methylenchlorid oder Ethylendichlorid als Lösungsmittel ist aus J.A.C.S, 1948, Seiten 3135-36 bekannt.

Auch die Veresterung von 5-Nitro-isophthalsäure mit Diazomethan (s. Indian Drugs & Pharmaceutical Industry, November/Dezember 1980, S. 35 ff.) befriedigt nicht, da Diazomethan sehr giftig und krebserregend ist und aufgrund seiner leichten Zersetzlichkeit im industriellen Maßstab nur unter sehr aufwendigen Vorsichtsmaßnahmen zu handhaben ist. Ein auf Diazomethan basierendes Herstellungsverfahren ist deshalb nicht wirtschaftlich durchzuführen. Es überrascht daher nicht, daß die gleichen Autoren in der gleichen Veröffentlichung ein weiteres Verfahren beschreiben, bei dem 5-Nitro-isophthalsäure zunächst mit Thionylchlorid ins Säurechlorid überführt wird, das dann nach Zwischenisolierung mit Methanol zum gewünschten Ester umgesetzt wird. Dabei müssen hohe Überschüsse an Reagenzien eingesetzt werden. Außerdem sind sehr lange Reaktionszeiten erforderlich (z.B. 48 h alleine für die Bildung des Säurechlorids). Die erhaltende Ware ist nicht rein, und muß wiederum mit den angeführten Nachteilen durch Umkristallisation gereinigt werden, so daß insgesamt drei Verfahrensschritte erforderlich sind. Diese aufwendige Synthese liefert das gewünschte Produkt auch nur in unbefriedigender Ausbeute. Eine wirtschaftliche Herstellung des gewünschten Produktes ist auch nach diesem Verfahren nicht möglich.

Als Verfahren zur Veresterung von Carbonsäuren ist auch die Veresterung mittels Dialkylsulfat bekannt. Entsprechend dem neueren Stand der Technik (EP-A 757 028) erfolgt sie in Anwesenheit eines wasserunlöslichen tertiären Amins und Wassers, gegebenenfalls in Anwesenheit eines wasserunlöslichen Lösungsmittels, unter Zugabe einer Base bei einem pH-Wert von 5 bis 12. Nachteilig bei diesem Verfahren ist die Verwendung der außerst canzerogenen Dialkylsulfate. 5-Nitro-isophthalsäure-di-methylester kann auf diese Weise offenbar nicht hergestellt werden. Das dürfte damit zusammenhängen, daß die gemäß der EP-A die Aufarbeitung der erhaltenen Ester in der Regel durch Destillation erfolgt (siehe dort S. 3, Zeile 19), was bei 5-Nitro-isophthalsäure-dimethylester nicht möglich ist, da schon sein Schmelzpunkt hoch ist (123°C) und Nitroverbindungen nicht beliebig thermisch belastbar sind.

Es wurde nun überraschenderweise ein Verfahren gefunden, das es gestattet, 5-Nitroisophthalsäure-di-C₁-C₄-alkylester aus 5-Nitro-isophthalsäure herzustellen, bei dem das gewünschte Produkt in einem einzigen Syntheseschritt in guter Ausbeute und guter Raum-Zeitausbeute in einer für pharmazeutische Zwecke geeigneten Reinheit von 99,9 % und mehr erhalten wird.

Das erfindungsgemäße Verfahren zur Herstellung von 5-Nitro-isophthalsäure-di-C₁-C₄-alkylestern ist dadurch gekennzeichnet, daß man 5-Nitro-isophthalsäure in einem Gemisch aus einem C₁-C₄-Alkylalkohol und einem aromatischen Kohlenwasserstoff, einem chloraromatischen oder einem aliphatischen Ether löst oder suspendiert, in Gegenwart einer starken Säure erwärmt, wobei sich eine zweite, wäßrige Phase bildet, nach Beendigung der Veresterungsreaktion aus der organischen Phase den gebildeten 5-Nitro-isophthalsäure-di-C₁-C₄-alkylester durch Abkühlen auskristallisiert und die wäßrige Phase vor oder nach der Abtrennung des 5-Nitro-isophthalsäure-di-C₁-C₄-alkylesters abtrennt.

Gegebenenfalls kann man die wäßrige Phase vor ihrer Abtrennung neutralisieren und/oder verdünnen und/oder die organische Phase vor der Abkühlung zur Kristallisation von 5-Nitro-isophthalsäure-di-C₁-C₄-alkylestem durch Extraktion reinigen.

Als C₁-C₄-Alkylalkohole werden vorzugsweise Methanol oder Ethanol eingesetzt. Bezogen auf 1 Mol 5-Nitro-isophthalsäure kann man beispielsweise 3 bis 8 Mol eines C₁-C₄-Alkylalkohols einsetzen.

Als aromatische Kohlenwasserstoffe, Chloraromaten und aliphatische Ether kommen z.B. Benzol, Toluol, Ethylbenzol, Cumol, Trimethylbenzole, Monochlorbenzol, Gemische isomerer Dichlorbenzole und Dibutylether. Bevorzugt sind Toluol und Xylol. Bezogen auf ein Volumenanteil C₁-C₄-Alkylalkohol kann man beispielsweise 0,2 bis 5 Volumenteile eines mit Wassers nicht oder nur teilweise mischbaren Lösungsmittels einsetzen.

Bei einer besonderen Ausführungsform des erfindungsgemäßen Verfahrens setzt man die 5-Nitro-isophthalsäure in wasserfeuchter und gegebenenfalls andere Säuren enthaltender Form und einen aromatischen Kohlenwasserstoff ein und entfernt vor der Zugabe des C₁-C₄-Alkylalkohols das eingebrachte Wasser durch azeotrope Destillation mit einem Teil des aromatischen Kohlenwasserstoffs.

Als starke Säuren kommen z.B. Schwefelsäure, Chlorwasserstoff (als Gas und als wäßrige Lösung), Methansulfonsäure, Toluolsulfonsäure und HSO₃-Gruppen enthaltende Ionenaustauscher in Frage. Bevorzugt sind Schwefelsäure und Salzsäure. Bezogen auf 5-Nitro-isophthalsäure kann man beispielsweise 1 bis 200 Gew.-% starke Säure einsetzen. Vorzugsweise beträgt diese Menge 10 bis 100 Gew.-%, insbesondere 30 bis 70 Gew.-%.

Das Erwärmen in Gegenwart einer starken Säure kann man beispielsweise auf Temperaturen im Bereich 50 bis 150°C durchführen. Bevorzugt sind Temperaturen im Bereich 70 bis 110°C, insbesondere im Bereich 80 bis 100°C. Bei zu tiefen Reaktionstemperaturen werden nicht nur die Reaktionszeiten sehr lange, es besteht dann auch die Gefahr, daß der gebildete 5-Nitro-isophthalsäure-di-C₁-C₄-alkylester zu früh auszukristallisieren beginnt.

Je nach der Zusammensetzung des Reaktionsgemisches kann man beispielsweise unter Normaldruck, gegebenenfalls bei Rückflußtemperatur, oder in geschlossenen Gefäßen unter erhöhtem Druck arbeiten.

Die Veresterung ist im allgemeinen nach einer Reaktionszeit von 2 bis 20 Stunden abgeschlossen. Vorzugsweise beträgt diese Zeit 3 bis 10 Stunden.

Vor oder nach der Abtrennung der wäßrigen Phase und vor dem Abkühlen zur Kristallisation des hergestellten Diesters kann man gegebenenfalls einen oder mehrere der folgenden Schritte durchführen:
- Filtration, z.B. zum Entfernen von festen Bestandteilen (z.B. sauren Ionenaustauschern),
- Abtrennung einer Mulmphase (falls vorhanden) und/oder
- Klärung des Reaktionsgemisches (mit einem Adsorbens wie Aktivkohle).

Wenn man hinreichend reine 5-Nitro-isophthalsäure einsetzt und ohne saure Ionenaustauscher gearbeitet hat, wird auch ohne diese Behandlungsschritte ein spezifikationsgerechter Diester erhalten.

Wenn die Bildung des 5-Nitro-isophthalsäure-di-C₁-C₄-alkylesters nicht vollständig verlaufen ist, enthält das nach der Reaktion vorliegende Reaktionsgemisch noch unumgesetzte 5-Nitro-isophthalsäure und/oder deren Mono-C₁-C₄-alkylester. Man kann vermeiden, daß diese zusammen mit dem gewünschten Diester ausfallen, wenn man das gesamte Reaktionsgemisch oder nur die organische Phase, gegebenenfalls nach einer Wäsche mit Wasser, mit einer wäßrigen Lösung einer Base behandelt. Zweckmäßigerweise führt man die erforderliche Phasentrennung und die Wäschen bei einer Temperatur durch, bei der der hergestellte 5-Nitro-isophthalsäure-di-C₁-C₄-alkylester noch nicht auskristallisiert. Bei der Base kann es sich z.B. um Alkalicarbonate, -hydrogencarbonate oder -hydroxide handeln. Bevorzugt sind wäßrige 5 bis 20 gew.-%ige Lösungen von Natrium- oder Kaliumcarbonat. Aus der wäßrigen Lösung einer Base können die abgetrennten Stoffe gegebenenfalls wiedergewonnen und einem nachfolgenden Ansatz zugefügt werden. Auf diese Weise ist eine Ausbeutesteigerung möglich. Es ist außerdem ein besonderer Vorteil des erfindungsgemäßen Verfahrens, daß man auch bei nicht vollständigem Umsatz ein hochreines Produkt erhalten kann.

Die Abkühlung der organischen Phase zur Kristallisation kann beispielsweise auf Temperaturen zwischen 25 und 0°C erfolgen. Es ist vorteilhaft während der Kristallisation zu rühren. Der Zusatz von Impfkristallen ist im allgemeinen nicht erforderlich.

Man kann auch so verfahren, daß man bei der Kristallisation keine vollständige Ausfällung der hergestellten Diesters anstrebt, sondern beispielsweise nur auf 20 bis 25°C abkühlt und die Mutterlauge dann einem nachfolgenden Ansatz zufügt. Das kann zu Ausbeutesteigerungen führen.

Das nach der Kristallisation von der Mutterlauge abgetrennte Produkt enthält i.a. geringe Anteile des verwendeten organischen Lösungsmittels. Es kann in dieser Form weiterverwendet werden. Gewünschtenfalls kann man das Lösungsmittel enthaltende Produkt noch waschen, z.B. mit Methanol, dann Wasser und gegebenenfalls trocknen. Man kann das Produkt auch in wasserfeuchter Form weiterverwenden.

Die Durchführung des erfindungsgemäßen Verfahrens kann z.B. auf folgende Weise erfolgen. 5-Nitro-isophthalsäure wird in einem Gemisch aus dem eingesetzten C₁-C₄-Alkylalkohol und einem unpolaren oder wenig polaren, aprotischen Lösungsmittel, unter Erwärmen gelöst und in Anwesenheit einer starken Säure einige Stunden erwärmt. Dabei bildet sich eine stöchiometrische Menge Wasser. Die zunächst homogene Lösung trennt sich dadurch in zwei Phasen. Die eine Phase enthält überwiegend Wasser und einen Teil der starken Säure sowie des C₁-C₄-Alkylalkohols, die andere Phase enthält neben dem organischen Lösungsgmittel den Rest des C₁-C₄-Alkylalkohols, gegebenenfalls 5-Nitro-isophthalsäure und gegebenenfalls Mono-C₁-C₄-alkylester und zwingend den gebildeten Dialkylester. Durch die Phasentrennung wird das Reaktionswasser aus der Phase entfernt, in der das gewünschte Hauptprodukt enthalten ist, und dadurch kann es nicht über das Veresterungsgleichgewicht bremsend auf die Reaktionsgeschwindigkeit und den Umsatz einwirken. Nach der gewünschten Reaktionszeit läßt man die Phasen sich trennen, trennt die wäßrige Phase ab (gegebenenfalls nach vorheriger Neutralisation), wäscht gegebenenfalls die noch warme organische Phase mit basischen wäßrigen Verbindungen frei von Säureresten, und läßt dann durch Abkühlen das gewünschte Produkt auskristallisieren.

Bei einer anderen Aufarbeitungsmethode gibt man das fertige Reaktionsgemisch auf Wasser, das soviel anorganische oder organische Base enthält, daß die Säure teilweise oder vollständig neutralisiert und die organische Phase abgekühlt wird. Gegebenenfalls ist die frei werdende Neutralisationswärme durch Kühlung abzuführen. Das erhaltene Produkt kann dann durch Filtration isoliert werden. Man kann auch die saure, wäßrige Phase neutralisieren, noch etwas zusätzliche Base (z.B. Soda oder Natriumbicarbonat) zugeben, abkühlen und die beiden flüssigen Phasen gleichzeitig durch Filtration von der festen Phase trennen. Die feste Phase ist dann das gewünschte Produkt.

Es können auch andere Durchführungsformen für das erfindungsgemäße Verfahren gewählt werden.

Der Hauptvorteil des erfindungsgemäßen Verfahrens liegt darin, daß das Produkt in hoher Ausbeute und der gewünschten Reinheit in einem Schritt erhalten wird und Zwischenisolierungen und/oder Umkristallisationen nicht erforderlich sind. Gegenüber dem Stand der Technik ist die Raum-Zeit-Ausbeute wesentlich erhöht. Es müssen keine hochcanzerogenen Substanzen gehandhabt und keine wasserunlösliche Basen zugegeben werden. Auch muß der pH-Wert während der Reaktion weder überwacht noch nachgestellt werden. Deshalb kann auf entsprechende apparative Ausrüstung verzichtet werden.

### Beispiele

### Beispiel 1

Es wurden 120 ml Methanol und 240 ml Toluol vermischt, 106 g 5-Nitro-isophthalsäure zugegeben und erwärmt, bis sich eine klare Lösung bildete. Man tropfte 33 ml 100 %ige Schwefelsäure zu und erhielt ein zweiphasiges Gemisch. Man erhitzte 4 Stunden zum Sieden. Danach wurde der Rührer angehalten, die untere (wäßrige) Phase abgelassen und die organische Phase einmal mit 100 ml heißem Wasser, dann einmal mit 100 ml heißer 10 %iger Natriumbicarbonat-Lösung gewaschen. Dann wurde die gewaschene organische Phase bis auf Raumtemperatur kalt gerührt. Dabei kristallisierte 5-Nitro-isophthalsäure-dimethylester in weißen Kristallen aus. Man filtrierte ab und wusch einmal mit 100 ml Methanol und einmal mit 100 ml Wasser. Man erhielt nach dem Trocknen 100 5-Nitro-isophthalsäure-dimethylester (Fp.: 123°C), der laut HPLC praktisch 100 %ig rein war, jedenfalls konnten Nebenkomponenten mit einem Gehalt von über 0,1 Gew.-% nicht gefunden werden.

### Beispiel 2

Man ging vor wie in Beispiel 1, kühlte aber die Suspension des Diesters bis auf 0°C ab. So wurden 100 g reiner 5-Nitro-isophthalsäure-dimethylester isoliert.

### Beispiel 3

Man ging vor wie in Beispiel 1, setzte aber die Mutterlauge aus dem Beispiel 1 anstelle von Toluol ein gab noch soviel frisches Toluol nach, daß die gleiche Einsatzrezeptur wie in Beispiel 1 erreicht wurde. Es wurden 113 g reiner 5-Nitro-isophthalsäure-dimethylester erhalten.

### Beispiel 4

Man ging vor wie in Beispiel 1, setzte aber 120 g Ethanol statt Methanol ein. Man isolierte 85 g reinen 5-Nitro-isophthalsäure-diethylester mit dem aus der Literatur bekannten Schmelzpunkt von 83,5°C (siehe Beilstein 9, 840).

### Beispiel 5

Man ging vor wie in Beispiel 1, setzte aber technisches Xylol statt Toluol ein. Man isolierte 97,5 g reinen 5-Nitro-isophthalsäure-dimethylester.

### Beispiel 6

Man ging vor wie in Beispiel 1, setzte aber Chlorbenzol statt Toluol ein. Dabei änderte sich das Phasenverhalten bei der Veresterung dergestalt, daß die abzutrennende wäßrige Phase leichter war als die den hergestellten Diester enthaltende organische Phase. Man isolierte 93,5 g reinen 5-Nitro-isophthalsäure-dimethylester.

### Beispiel 7

Man legte 210 g Di-isobutylether und 19 g Methanol vor, löste darin unter Erwärmen 21 g 5-Nitro-isophthalsäure und tropfte unter Rühren 7 ml konzentrierte Schwefelsäure zu. Dann erhitzte man 5 Stunden zu leichtem Sieden, trennte die untere dunkle Phase ab und goß die obere helle Phase in eine Vorlage aus 20 g Natriumcarbonat und 20 g Natriumbicarbonat gelöst in 1 l Wasser. Man rührte kalt, saugte ab und wusch mit Wasser und wenig Methanol. Man erhielt 18,5 g reinen 5-Nitro-isophthalsäure-dimethylester. Aus der Mutterlauge wurde der Diisobutylether durch Phasentrennung fast vollständig wiedergewonnen.

### Beispiel 8

Man legte 220 ml Toluol in 80 ml Methanol vor, gab 106 g 5-Nitro-isophthalsäure zu und tropfte unter Erwärmen 8 ml konzentrierte Schwefelsäure zu. Man erhitzte 8 Stunden auf 80°C, neutralisierte mit 10 gew.-%iger wäßriger Soda-Lösung und rührte kalt. Man saugte ab, wusch dreimal mit je 100 ml 10 gew.-%iger wäßriger Sodalösung, dann zweimal mit Wasser und trocknete. Man erhielt 87,5 g reinen 5-Nitro-isophthalsäure-dimethylester.

### Beispiel 9 (zum Vergleich)

Zu einer Lösung von 195 g Isophathalsäure-dimethylester in 320 ml 100 gew.-%iger Schwefelsäure tropfte man bei einer Temperatur von 10°C unter Rühren eine Mischung aus 100 g Salpetersäure und 302 g Schwefelsäure und rührte noch 3 Stunden bei 20 bis 25°C nach. Man gab auf 1 600 g Eis/Wasser-Gemisch und kühlte dabei die Vorlage so, daß die Temperatur 10°C nicht überstieg. Man rührte noch 1 Stunde nach und filtrierte über ein säurefestes Filter. Man wusch mit viel Wasser säurefrei und trocknete das Produkt im Vakuum.

Man erhielt 230 g Produkt, das 98,0 Gew.-% 5-Nitro-isophthalsäure-dimethylester, 1,0 Gew.-% 4-Nitro-isophthalsäure-dimethylester und 0,3 Gew.-% 5-Nitro-isophthalsäure-monomethylester enthielt.

## Patentansprüche

1. Verfahren zur Herstellung von 5-Nitro-isophthalsäure-di-C₁-C₄-alkylestem, dadurch gekennzeichnet, daß man 5-Nitro-isophthalsäure in einem Gemisch aus einem C₁-C₄-Alkylalkohol und einem aromatischen Kohlenwasserstoff, einem Chloraromaten oder einem aliphatischen Ether löst oder suspendiert, in Gegenwart einer starken Säure erwärmt, wobei sich eine zweite, wäßrige Phase bildet, nach Beendigung der Veresterungsreaktion aus der organischen Phase den gebildeten 5-Nitro-isophthalsäure-di-C₁-C₄-alkylester durch Abkühlen auskristallisiert und die wäßrige Phase vor oder nach der Abtrennung des 5-Nitro-isophthalsäure-di-C₁-C₄-alkylesters abtrennt.

2. Verfahren nach Anspruch 1, dadurch gekennzeichnet, daß man bezogen auf 1 Mol 5-Nitro-isophthalsäure 3 bis 8 Mol eines C₁-C₄-Alkylalkohols, einsetzt.

3. Verfahren nach Ansprüchen 1 bis 2, dadurch gekennzeichnet, daß man bezogen auf ein Volumenteil C₁-C₄-Alkylalkohol 0,2 bis 5 Volumenteile eines mit Wasser nicht oder nur teilweise mischbaren Lösungsmittels einsetzt.

4. Verfahren nach Ansprüchen 1 bis 3, dadurch gekennzeichnet, daß man als starke Säuren Schwefelsäure, Chlorwasserstoff (als Gas oder als wäßrige Lösung), Methansulfonsäure, Toluolsulfonsäure oder HSO₃-Gruppen enthaltende Ionenaustauscher in einer Menge von 1 bis 200 Gew.-%, bezogen auf 5-Nitro-isophathalsäure, einsetzt.

5. Verfahren nach Ansprüchen 1 bis 4, dadurch gekennzeichnet, daß man das Erwärmen in Gegenwart einer starken Säure auf Temperaturen im Bereich 50 bis 150°C durchführt.

6. Verfahren nach Ansprüchen 1 bis 5, dadurch gekennzeichnet, daß die Reaktionszeit zwischen 2 bis 20 Stunden beträgt.

7. Verfahren nach Ansprüchen 1 bis 6, dadurch gekennzeichnet, daß man das gesamte ausreagierte Reaktionsgemisch oder nur die organische Phase mit einer wäßrigen Lösung einer Base behandelt.

8. Verfahren nach Ansprüchen 1 bis 7, dadurch gekennzeichnet, daß die Abkühlung der organischen Phase zur Kristallisation auf Temperaturen zwischen 25 und 0°C erfolgt.

9. Verfahren nach Ansprüchen 1 bis 8, dadurch gekennzeichnet, daß man die organische Phase zur Kristallisation auf 20 bis 25°C abkühlt und die Mutterlauge dann einem nachfolgenden Ansatz zufügt.

## Claims

1. Process for preparing di-C₁-C₄-alkyl 5-nitro-isophthalates, characterized in that 5-nitroisophthalic acid is dissolved or suspended in a mixture of a C₁-C₄-alkyl alcohol and an aromatic hydrocarbon, a chlorinated aromatic compound or an aliphatic ether heated in the presence of a strong acid, thus forming a second, aqueous phase, the di-C₁-C₄-alkyl 5-nitro-isophthalate formed is, after the esterification reaction has ended, crystallized out from the organic phase by cooling and the aqueous phase is removed before or after the di-C₁-C₄-alkyl 5-nitro-isophthalate is separated off.

2. Process according to Claim 1, characterized in that 3 to 8 mol of a C₁-C₄-alkyl alcohol are employed, based on 1 mol of 5-nitro-isophthalic acid.

3. Process according to Claims 1 and 2, characterized in that 0.2 to 5 parts by volume of a water-immiscible or only partially water-miscible solvent are employed, based on one part by volume of C₁-C₄-alkyl alcohol.

4. Process according to Claims 1 to 3, characterized in that the strong acids employed are sulphuric acid, hydrogen chloride (as a gas or as an aqueous solution), methanesulphonic acid, toluenesulphonic acid or ion exchangers containing HSO₃ groups in an amount of 1 to 200% by weight, based on 5-nitroisophthalic acid.

5. Process according to Claims 1 to 4, characterized in that the heating in the presence of a strong acid is carried out to temperatures in the range from 50 to 150°C.

6. Process according to Claim 1 to 5, characterized in that the reaction time is between 2 and 20 hours.

7. Process according to Claims 1 to 6, characterized in that the entire reacted reaction mixture or just the organic phase is treated with an aqueous solution of a base.

8. Process according to Claims 1 to 7, characterized in that the organic phase is cooled for crystallization to temperatures between 25 and 0°C.

9. Process according to Claims 1 to 8, characterized in that the organic phase is cooled to 20 to 25°C for crystallization and the mother liquor is then added to a subsequent reaction.

## Revendications

1. Procédé pour la préparation des 5-nitro-isophtalates de di(alkyle en C₁-C₄) caractérisé en ce que l'on dissout l'acide 5-nitro-isophtalique ou on le met en suspension dans un mélange d'un alcanol en C₁-C₄ et d'un hydrocarbure aromatique, d'un hydrocarbure aromatique chloré ou d'un éther aliphatique, on chauffe en présence d'un acide fort, ce qui provoque la formation d'une seconde phase aqueuse, on fait cristalliser le 5-nitro-isophtalate de di(alkyle en C₁-C₄) formé de la phase organique après la réaction d'estérification par refroidissement et on sépare la phase aqueuse avant ou après avoir séparé le 5-nitro-isophtalate de di(alkyle en C₁-C₄).

2. Procédé selon la revendication 1, caractérisé en ce que l'on met en oeuvre de 3 à 8 mol d'un alcanol en C₁-C₄/mole de l'acide 5-nitro-isophtalique.

3. Procédé selon les revendications 1 à 2, caractérisé en ce que l'on utilise de 0,2 à 5 volumes d'un solvant non miscible à l'eau ou partiellement miscible à l'eau par volume de l'alcanol en C₁-C₄.

4. Procédé selon les revendications 1 à 3, caractérisé en ce que l'on utilise en tant qu'acides forts l'acide sulfurique, le chlorure d'hydrogène (à l'état gazeux ou à l'état de solution aqueuse), l'acide méthane sulfonique, l'acide toluène sulfonique ou un échangeur d'ions à groupes HSO₃, en quantité de 1 à 200 % du poids de l'acide 5-nitro-isophtalique.

5. Procédé selon les revendications 1 à 4, caractérisé en ce que l'on chauffe en présence d'un acide fort à des températures dans l'intervalle de 50 à 150°C.

6. Procédé selon les revendications 1 à 5, caractérisé en ce que la durée de réaction est de 2 à 20 h.

7. Procédé selon les revendications 1 à 6, caractérisé en ce que l'on traite par une solution aqueuse d'une base tout le mélange de réaction final ou uniquement la phase organique.

8. Procédé selon les revendications 1 à 7, caractérisé en ce que, pour la cristallisation, on refroidit la phase organique à des températures allant de 25 à 0°C.

9. Procédé selon les revendications 1 à 8, caractérisé en ce que l'on refroidit la phase organique pour cristallisation à des températures de 20 à 25°C et on recycle les liqueurs mères dans une opération consécutive.
